# EUROPEAN PATENT SPECIFICATION

(11) **EP 0 800 501 B1**
(45) Date of publication and mention of the grant of the patent: **06.10.1999**
(21) Application number: 95944255.9
(22) Date of filing: 28.12.1995
(51) Int. Cl.: C07C 51/14, C07C 53/128

(54) **PRODUCTION OF TRIALKYLACETIC ACIDS USING A SOLID CATIONIC RESIN CATALYST SYSTEM**
HERSTELLUNG VON TRIALKYLCARBONSÄUREN UNTER VERWENDUNG EINES FESTEN SAUREN HARZKATALYSATORSYSTEMS
PRODUCTION D'ACIDES TRIALKYLACETIQUES AU MOYEN D'UN CATALYSEUR SOLIDE DE RESINE CATIONIQUE

(30) Priority: 28.12.1994 US 365390
(43) Date of publication of application: 15.10.1997
(73) Proprietor: EXXON CHEMICAL PATENTS INC., Baytown, TX 77520-5200 (US)
(72) Inventor: YOUNG, David, Alexander, Shoreline, WA 98155 (US); CONNOR, Thomas, H., Baton Rouge, LA 70809 (US); SCHLOSBERG, Richard, Henry, Bridgewater, NJ 08807 (US); EMERT, Jacob, Brooklyn, NY 11218 (US); DIANA, William, Daniel, Belle Mead, NJ 08502 (US); GORDA, Keith, R., Little York, NJ 08834 (US); CUSUMANO, Joseph, Victor, Watchung, NJ 07060 (US)
(74) Representative: Dew, Melvyn John
(86) International application number: US9516956
(87) International publication number: WO9620154

(56) References cited:
- EP-A- 0 249 976
- EP-A- 0 296 275
- EP-A- 0 347 939
- WO-A-92/18452
- WO-A-92/18592
- US-A- 5 250 726

## Description

This invention deals with the production of trialkylacetic acids from branched olefins, particularly isononene, by reacting said branched olefins with carbon monoxide using a solid cationic resin capable of providing a proton acid as catalyst.

Trialkylacetic acids have found a variety of uses in the chemical industry. By way of example, neodecanoic acid is converted to metal salts and acts as a paint thinner. It is derivatized to vinyl esters and glycidyl esters for use in coating applications. Similarly, neopentanoic (pivalic) acid has found wide use in the production of agrichemicals, specialty chemicals and pharmaceuticals.

Trialkylacetic acids have been produced by various processes using liquid catalysts, such as BF₃·2H₂O, HF, H₃PO₄, BF₃·H₂O; H₂SO₄; mixtures of BF₃·2H₂O with H₂SO₄ or H₃PO₄. Such technology is described in US-A-3,068,256 (Roming), EP-A-0 296 275 (Idemitsu) and in other patent literature. Such processes, however, have disadvantages. A large inventory of corrosive acid is used. These processes require expensive materials of construction and relatively difficult catalyst separation and recovery steps.

A variety of other processes are described in the patent literature.

EP-A-0 249 976 (BASF) describes an aqueous system for reacting olefins with carbon monoxide and H₂O in the presence of acidic zeolites as catalysts. Zeolites of the pentasil type are preferred. Olefin conversion is preferably conducted in the gas phase. In one embodiment its zeolitic material is treated with acids.

In contrast to the BASF patent which uses inorganic metallic oxide zeolites having relatively low acid strength, the present process employs organic resins having an acidity equivalent to or greater than a 65 % sulfuric acid solution and uses a non-aqueous reaction system.

Mitsubishi Chemical Japanese Patent 62187424 describes a process for the carboxylation of olefins by reacting the olefins with a hydroxyl compound selected from water, alcohol and carboxylic acid in the presence of a palladium catalyst and organic phosphine. A sulfonic acid ion-exchange resin is used as an accelerator.

EP-A-0347 939 (Nippon Petrochemicals) describes a process for the carboxylation of olefins by reacting the olefins with carbon monoxide and water in the presence of a transition metal complex catalyst such as a complex of palladium. Hydrogen chloride or boron trifluoride may be used as the accelerator.

US-A-5,250,726 (Du Pont) describes the preparation of 3-pentenoic acid by the reaction of butadiene and carbon monoxide in the presence of a rhodium catalyst, a sulfonic acid catalyst, water and a carboxylic acid solvent.

Two recent patents to Catalytica, Inc. are also noted. US-A-5,227,521 discloses a process for producing trialkylacetic acids from branched olefins by reaction with carbon monoxide and water in the presence of Lewis acids. US-A-5,241,112 similarly reacts branched olefins with carbon monoxide and water but uses a solid acid catalyst referred to as Molecular Engineered Layered Structures (MELS). It preferably is a compound comprising sulfonic acid and phosphonic acid groups where the phosphonic acid groups are covalently bonded to a polymeric chain.

It is noted that essentially all the foregoing processes employ aqueous reaction conditions. Aqueous systems in general have the disadvantage of presenting a corrosive reaction environment and acting as a catalyst poison by weakening the acidity of the catalyst. Non-aqueous system are more selective in permitting alternative products, such as esters, to be directly produced.

The present invention relates to a non-aqueous process for producing trialkylacetic acids from corresponding branched olefins by reacting said olefins with carbon monoxide in the presence of a solid cation exchange resin catalyst capable of forming a cation with the feed olefin and which typically is a cationic resin having sulfuric acid on its surface. The reaction takes place in a non-aqueous environment. The cation exchange resin has a 65 wt. percent, preferably 70%, or greater sulfuric acid strength equivalent.

The present process finds application for converting branched olefins to trialkylacetic acids represented by the following formula.

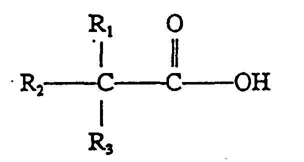

Where each R group is an alkyl group has 1 to 10 carbons. Typically, the total number of carbon atoms in the molecule is 5 to 19, preferably 5 to 13.

The branched or unbranched olefins used as feed reactant have the general formula:

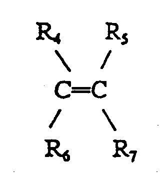

Where each R is an alkyl group having 1 to 15 carbons, or alternatively R₄ and/or R₅ can be hydrogen. Typically the total number of carbon atoms, in the olefin is 4 to 18, preferably 4 to 12.

The foregoing branched or unbranched olefins are reacted with CO in the presence of the instant catalyst system in a non-aqueous reaction system.

The reaction conditions are summarized in the following table.

| **CONDITIONS** | **BROAD RANGE** | **PREFERRED RANGE** |
|---|---|---|
| Reaction temperature °C | 25 - 120 | 50 - 120 |
| Carbon Monoxide Pressure, MPag (psig) | 3.45 - 20.7 (500 - 3,000) | 1,000 - 1,500 |
| Resin acid strength, equivalent to sulfuric acid | 65 wt. percent or greater Sulfuric acid | 70 wt. percent or greater Sulfuric acid |

Sufficient catalyst is used to provide at least a molar equivalent of acidic (proton) group to the olefin.

The catalyst of the present invention is a solid resin showing strong-acid behavior. It is preferably selected from the group consisting of sulfonated copolymers of styrene and DVB (divinylbenzene) and phenol or phenolic based resins. In either case, the resin is treated to give a sulfonic acid cation - exchange resin capable of providing sufficient protons (resin having an acid strength equivalent to at least 65 weight percent sulfuric acid) for the reaction to proceed to yield trialkylacetic acids.

### EXAMPLES

The following examples illustrate the synthesis of trialkylacetic acids, particularly neodecanoic acid, from branched olefins such as isononene, by the present catalyst system as well as the need to maintain non-aqueous conditions during the synthesis reaction.

In each example a cation exchange styrene divinylbenzene resin bead produced by Rohm and Haas Corporation, Philadelphia, PA, and sold under the name Amberlyst 15 was used. It is a well-known strongly acidic cation exchange resin having sulfonic groups attached to its backbone and sold in bead form. Amberlyst 15 is treated by the manufacturer with sulfuric acid to convert it into the acid form. The presence of inorganic acid residue on the ion exchange beads could serve as a catalyst for the production of trialkylacetic acids (neo acids). In order not to give a false positive result by the possible presence of free sulfuric acid, the resin was carefully extracted with water (50ml of water per 100g of received resin) to ensure there was no measurable inorganic residue present. The resin was then separated and dried.

The feed olefin in each case was isononene, a commercially available (Exxon Chemical Company) branched C₉ olefin made by the acid catalyzed oligomerization of propylene. It contains a large number of branched internal olefin isomers.

A two liter rocking autoclave was used in each example with carbon monoxide serving to pressurize the reaction mixture. Heptane was used as solvent and other than Example 2 the reaction was conducted under substantially aqueous free conditions.

### EXAMPLE 1

A 289.9 g sample of isononene dissolved in 246.0 g n-heptane solvent was charged to the two liter rocking autoclave with 163.9 g of dry Amberlyst 15 resin beads obtained from Rohm and Hass Corp. The autoclave was pressurized to 6.9 MPag (1000 psig) with carbon monoxide, heated with rocking to 155°C and the pressure then adjusted to 10.3 MPag (1500 psig) with carbon monoxide. These reaction conditions were maintained for six hours.

The autoclave was then cooled to room temperature and vented. The recovered product mixture was stirred with 500 ml water for three hours to separate reaction product. The organic phase was then separated from the aqueous phase and resin beads and analyzed by gas chromatography using known standards and calculated response factors. In all examples a gas chromatographic system using a Hewlett/Packard 5890 series 11 with an FID, HP-7673 autoinjector and HP plotter integrator or equivalent was used together with a column injection system. A CIS/CALS integrating computer system was used. A 30 meter x.53 mm ID FFAP column with about a 1 micron film was employed. Typically, an initial injector temperature of 123° and detection temperature of 280°C was used. The oven temperature ran from 120 to 240°C at increases of 10°C/min.

The organic phase was found to consist of the following:
29 weight % unreacted isononene
42 weight % neodecanoic acid (mixed isomers)
26 weight % isooctadecenes (mixed isomers)
3 weight % C₂₇ olefins (mixed isomers)

The amount of neodecanoic acid produced corresponded, on a molar basis, to 92 % of the theoretical active acid sites available on the resin beads as reported by the manufacturer.

### EXAMPLE 2

Example 1 was repeated with water present during the reaction. The following feeds were charged to the same two liter autoclave: 286.2 g isononene, 247.6 g n-heptane, 143.0 g Amberlyst 15 resin beads, and 12.3 g water. The reaction conditions were the same as Example 1 with the exception that a reaction temperature of 115°C was used. The recovered product was treated as above.

Gas chromatographic analysis gave no evidence of trialkylacetic acid product formation. This illustrates that the present process must be conducted with these resin catalysts in a non-aqueous system.

### EXAMPLE 3

Example 1 was repeated at a lower reaction temperature. The following feed materials were charged to the two liter autoclave: 290.0 g isononene, 249.0 g n-heptane, and 137.2 g Amberlyst 15 resin beads. The reaction conditions were the same as Example 1 with the exception that a reaction temperature of 100°C was used.

The recovered product mixture was filtered to separate the organic phase from the resin beads. The latter were rinsed with 100 cc n-heptane and returned to the autoclave for use in Example 4. The n-heptane rinse was combined with the organic phase and analyzed as per Example 1. The following results were obtained:
38 weight % unreacted isononene
26 weight % neodecanoic acid (mixed isomers)
35 weight % isooctadecene (mixed isomers)
1 weight % C₂₇ olefins (mixed isomers)

The higher temperature of Example 1 gave somewhat better trialkylacetic acid product formation.

### EXAMPLE 4

Example 3 was repeated using the Amberlyst 15 resin beads recovered from Example 3 and the following feeds: 290.0 g isononene and 249.0 g n-heptane. The reaction conditions and product recovery were the same as given in Example 3. Analysis of the product gave the following results:
78 weight % unreacted isononene
5 weight % neodecanoic acid (mixed isomers)
17 weight % isooctadecene (mixed isomers)
a trace of C₂₇ olefins (mixed isomers)

This example illustrates the depletion of the active sites of the cation exchange resin and its ability to serve, as a `catalyst for converting branched olefins to trialkylacetic acids.

## Claims

1. A process for the production of trialkylacetic acids comprising the steps of contacting under non-aqueous conditions a branched or unbranched olefin having the general formula: 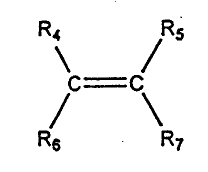 wherein each R is an alkyl group of 1 to 15 carbons, or alternatively R₄ and/or R₅ is hydrogen with carbon monoxide and a cationic resin having sufficient acid groups to provide requisite protons for conversion of said olefin and carbon monoxide to trialkylacetic acids, wherein said cationic resin catalyst has an acidity at least equivalent to that of a 65 weight percent sulfuric acid.

2. The process of claim 1 wherein said reaction is conducted at a temperature in the range of 25-120°C.

3. The process of any of the preceding claims wherein a carbon monoxide pressure of 3.45-20.7 MPag (500-3000 psig) is used during the reaction.

4. The process of any of the preceding claims where said cationic resin is selected from the group consisting of sulfonated copolymers of styrene and divinylbenzene; phenol and phenolic based resins.

5. The process of any of the preceding claims where said feed olefin is isononene and the trialkylacetic acid product is neodecanoic acid.

## Patentansprüche

1. Verfahren zur Herstellung von Trialkylessigsäuren, bei dem in Stufen unter nicht-wäßrigen Bedingungen ein verzweigtes oder unverzweigtes Olefin mit der allgemeinen Formel 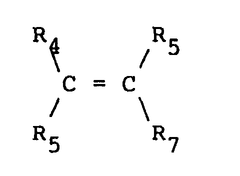 in der jedes R eine Alkylgruppe mit 1 bis 15 Kohlenstoffatomen ist oder alternativ R₄ und/oder R₅ Wasserstoff sind, mit Kohlenmonoxid und einem kationischen Harz mit ausreichend Säuregruppen, um die erforderlichen Protonen zur Umwandlung des Olefins und Kohlenmonoxids zu Trialkylessigsäuren bereitzustellen, kontaktiert wird, wobei der kationische Harzkatalysator eine Acidität hat, die mindestens der von 65 Gew.% Schwefelsäure entspricht.

2. Verfahren nach Anspruch 1, bei dem die Reaktion bei einer Temperatur im Bereich von 25 bis 120°C durchgeführt wird.

3. Verfahren nach einem der vorhergehenden Ansprüche, bei dem während der Reaktion ein Kohlenmonoxidüberdruck von 3,45 bis 20,7 MPa (500 bis 3000 psi) verwendet wird.

4. Verfahren nach einem der vorhergehenden Ansprüche, bei dem das kationische Harz ausgewählt ist aus der Gruppe bestehend aus sulfonierten Copolymeren von Styrol und Divinylbenzol, Phenol und Harzen auf Phenolbasis.

5. Verfahren nach einem der vorhergehenden Ansprüche, bei dem das Einsatzolefin Isononen ist und das Trialkylessigsäureprodukt Neodecansäure ist.

## Revendications

1. Procédé de production d'acides trialkylacétiques, comprenant les étapes de mise en contact, dans des conditions non aqueuses, d'une oléfine ramifiée ou non ramifiée répondant à la formule générale : 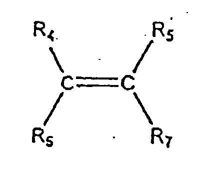 dans laquelle chaque R est un groupe alkyle ayant 1 à 15 atomes de carbone, ou bien en variante, R₄ et/ou R₅ représentent de l'hydrogène, avec du monoxyde de carbone et une résine cationique portant suffisamment de groupes acide pour fournir les protons nécessaires à la transformation de cette oléfine et du monoxyde de carbone en acides trialkylacétiques, cette résine cationique utilisée comme catalyseur ayant une acidité qui équivaut au moins à celle d'un acide sulfurique à 65 % en poids.

2. Procédé suivant la revendication 1, dans lequel la réaction en question est conduite à une température comprise dans la plage de 25 à 120°C.

3. Procédé suivant l'une quelconque des revendications précédentes, dans lequel on utilise pendant la réaction une pression manométrique de monoxyde de carbone de 3,45 à 20,7 MPag (500 à 3000 lg/in²).

4. Procédé suivant l'une quelconque des revendications précédentes, dans lequel la résine cationique est choisie dans le groupe consistant en copolymères sulfonés de styrène et de divinylbenzène ; résines phénoliques et résines à base phénolique.

5. Procédé suivant l'une quelconque des revendications précédentes, dans lequel l'oléfine de la charge est l'isononène et l'acide trialkylacétique obtenu comme produit est l'acide néodécanoïque.
